# EUROPÄISCHE PATENTSCHRIFT

(11) **EP 2 174 590 B1**
(45) Veröffentlichungstag und Bekanntmachung des Hinweises auf die Patenterteilung: **23.03.2016**
(21) Anmeldenummer: 09012408.2
(22) Anmeldetag: 30.09.2009
(51) Int. Cl.: A61B 5/113, A61B 5/08, A61B 5/00, G01S 11/02

(54) **Vorrichtung und Verfahren zum Erfassen einer Atmung eines Lebewesens**
Method and device for recording the breathing of an animal
Dispositif et procédé de détermination d'une respiration d'un être vivant

(30) Priorität: 07.10.2008 DE 102008050640; 06.11.2008 DE 102008056252
(43) Veröffentlichungstag der Anmeldung: 14.04.2010
(73) Patentinhaber: Fraunhofer-Gesellschaft zur Förderung der angewandten Forschung e.V., 80686 München (DE)
(72) Erfinder: Tobola, Andreas, 91334 Hemhofen (DE); Ershov, Sergey, 91054 Erlangen (DE); Wissendheit, Uwe, 91052 Erlangen (DE); Couronné, Robert, 91058 Erlangen (DE)
(74) Vertreter: Hersina, Günter

(56) Entgegenhaltungen:
- EP-A1- 1 661 511
- DE-A1-102005 059 687
- US-A- 3 911 899
- US-A1- 2005 084 075
- US-A1- 2008 103 702

## Beschreibung

Die vorliegende Erfindung bezieht sich auf Vorrichtungen und Verfahren zum Erfassen einer Atmung oder Atmungsanstrengung von Lebewesen, z. B. von Menschen oder Tieren.

Mit der Atmungsanstrengung wird die Variation des Körperumfangs an mindestens einer Stelle am Rumpf, z. B. am Brustkorb, eines Lebewesens bezeichnet. Typischerweise wird die Atmungsanstrengung beim Menschen an zwei Stellen des Rumpfes, an Abdomen bzw. Bauch und Thorax bzw. Brustkorb, gemessen und als Signal abgebildet oder gespeichert.

Aus dem Rohsignal der Atmungsanstrengung werden Vitalparameter, wie Atmungsfrequenz, Atmungsamplitude und Atmungsvolumen berechnet, die wertvolle Informationen über den Zustand des Menschen liefern. Im Allgemeinen kann mittels der Atmungsanstrengung, alleine oder in Kombination mit anderen Vitalparametern, eine Aussage über den Zustand eines Menschen bzw. Patienten getroffen werden.

Herkömmliche Verfahren beruhen auf der Messung der Atmungsanstrengung mittels Bänder, die um eine Körperpartie angelegt werden. Die Methode, mit der die Atmungsanstrengung ermittelt wird, lässt sich in zwei Klassen einordnen. Zum einen sind dies belastungsfreie Verfahren und zum anderen solche, die eine gewisse Spannung der Bänder erfordern. Als Beispiel für ein belastungsfreies Verfahren ist die induktive Plethysmographie zu nennen, bei der die Bänder locker um die ausgewählten Körperpartien angebracht werden. Unabhängig von der Messmethode können solche Bänder unter der Kleidung getragen werden, müssen jedoch darunter mittels Kabeln an eine Signalaufbereitungselektronik angeschlossen werden.

Die Patentschrift US 5,825,293 beschreibt eine Methode zur Überwachung, im Englischen auch als "Monitoring" bezeichnet, der Atmungsfrequenz mittels Detektierung der Veränderungen des Magnetfeldes eines Permanentmagneten, der am Körper des Patienten angebracht ist.

Die Patentveröffentlichung US 3 911 899 A offenbart eine Atmungsüberwachungsmethode, bei der eine Transmitterspule und eine Empfängerspule auf gegenüberliegenden Seiten des Brustkorps angeordnet sind, um basierend auf durch die Atmung hervorgerufenen Änderungen des Abstands zwischen der Senderspule und der Empfängerspule ein Atmungssignal zu erzeugen, wobei die Atmungsbewegung eine Änderung des von der Empfängerspule erfassten magnetischen Feldes bewirkt. Ein entsprechendes System zur Atmungserfassung zeigt auch die weitere Signalverarbeitung einschließlich des Atmungsratenmeters und der Alarmvorrichtung. Die zwei Spulen werden beispielsweise bei einer Frequenz von 4096 Hz betrieben.

Die Patentveröffentlichung EP 1 661 511 A1 beschreibt ein System zum Erfassen einer biologischen Information (zumindest 2 Informationen aus einer Herzschlaginformation, einer Körperbewegungsinformation und einer Atmungsinformation) einer Person, nämlich des Fahrers eines Kraftfahrzeugs. Dabei ist ein Körpersensor in Form eines piezoelektrischen Kabelsensors als Beschleunigungserkennungsvorrichtung mäanderförmig in einen Sicherheitsgurt integriert, der eine Vibration des Körpers der Person misst. Das Beispiel weist ferner einen Autosensor auf, der eine Vibration des Kraftfahrzeugs misst, um die Vibration des Kraftfahrzeugs, die sich störend über die Messung der Körpervibrationen (für die biologischen Informationen) überlagert, herausrechnen zu können.

Die Patentveröffentlichung US 2005/084075 A1 offenbart eine Aktivitätsüberwachungsvorrichtung mit einem Permanentmagnet, der in einer Tasche eines Kleidungsstücks angebracht ist, und sich entsprechend der Bewegung der Brust der Person während der Atmung bewegt. Ein Magnetfeldsensor, beispielsweise ein Halleffektsensor ist diesem Permanentmagneten gegenüber in einer festen Position in der Nähe zu dem Nutzer, beispielsweise neben dem Bett des Nutzers, angeordnet.

Ein Nachteil der Atmungsbänder ist, dass diese vor der Messung an dem Körper angelegt und an eine Signalaufbereitungselektronik angeschlossen werden müssen.

Ein Nachteil der Methode, bei der die Magneten in einem Kleidungsstück integriert sind, ist, dass der jeweilige Patient ein solches mit einem Magneten versehenes Kleidungsstück trägt bzw. vorher anziehen muss.

### Zusammenfassung

Ein Ausführungsbeispiel der vorliegenden Erfindung schafft eine Vorrichtung zum Erfassen einer Atmung eines Lebewesens mit folgenden Merkmalen: einem aktiven Sender, der ausgebildet ist, um ein magnetisches oder elektromagnetisches Feld zu erzeugen; und einem Messfühler, der an dem Rumpf des Lebewesens angeordnet ist und ausgebildet ist, um ein Signal bereitzustellen, das von dem magnetischen oder elektromagnetischen Feld und von einer durch die Atmung des Lebewesens bedingten Abstandsänderung zwischen Sender und Messfühler abhängt.

Ein weiteres Ausführungsbeispiel der vorliegenden Erfindung schafft ein Verfahren zum Erfassen einer Atmung eines Lebewesens mit folgenden Schritten: Erzeugen eines magnetischen oder elektromagnetischen Feldes mittels eines aktiven Senders; und Bereitstellen eines Signals mittels eines Messfühlers, der am Rumpf des Lebewesens angeordnet ist, wobei das Signal von dem magnetischen oder elektromagnetischen Feld und von einer durch die Atmung des Lebewesens bedingten Abstandsänderung zwischen dem aktiven Sender und dem Messfühler abhängt.

Ausführungsbeispiele des Verfahrens und der Vorrichtung ermöglichen die Durchführung von Messungen der Atmungsanstrengung mit geringerer Beeinträchtigung des Menschen bzw. allgemein eines Lebewesens.

Bei Ausführungsbeispielen des Verfahrens und der Vorrichtung sind die Vorrichtungen zur Messung der Atmungsanstrengung, der aktive Sender und der Messfühler, in Umgebungsobjekten integriert, mit denen ein Mensch bzw. allgemein ein Lebewesen in unmittelbaren Kontakt kommt. Beispiele für derartige Umgebungsobjekte sind die Rückenlehne eines Autositzes und der Sicherheitsgurt.

Ausführungsbeispiele der Vorrichtung und des Verfahrens setzen kein Anbringen sogenannter Anwendungsteile, d. h. Teile, die am Patientenkörper angebracht bzw. befestigt werden (z. B. Atmungsbänder), um die Körper herum zur Messung der Atmungsanstrengung voraus.

Ausführungsbeispiele der Vorrichtung und des Verfahrens können ohne weiteres in so genannte "Umgebungsobjekte" des Menschen, z. B. Bedienelemente, Sitze, Liegen, Sicherheitsgurte, Lenkräder etc., so integriert werden, dass diese von außen unauffällig bis nicht erkennbar sind. Somit können die Messungen für den Menschen bzw. das Lebewesen unbemerkt durchgeführt bzw. ohne zusätzlichen Aufwand von Seiten des Menschen, wie z. B. das Anbringen der Atmungsbänder oder Anziehen entsprechender Kleidungsstücke, durchgeführt werden.

Ausführungsbeispiele der Vorrichtung und des Verfahrens können ferner an solchen Stellen bzw. in solchen Fällen verwendet werden, an bzw. bei denen Atmungsbänder oder spezielle Kleidungsstücke aufgrund hygienischer sowie technischer Probleme oder bei Problemen mit Patientenakzeptanz nicht verwendet werden können.

In Ausführungsbeispiele der vorliegende Erfindung ist der aktive Sender ausgebildet, durch Änderung beispielsweise der Frequenz oder Amplitude des magnetischen oder elektromagnetischen Feldes die Vorrichtung zum Erfassen an sich ändernde Messbedingungen anzupassen. So kann z.B. die Sendeleistung abhängig von dem mittleren Abstand zwischen dem aktiven Sender und dem Meßfühler, der ein Maß für die Dicke des Rumpfs oder Brustkorbs ist, erhöht oder reduziert werden, um eine zuverlässige Messung zu ermöglichen, gleichzeitig aber z.B. den Stromverbrauch gering zu halten. Somit wird unabhängig vom Rumpfumfang des Lebewesens eine optimale Messung ermöglicht.

In weiteren Ausführungsbeispielen der Vorrichtung und des Verfahrens, bei denen nicht alle Komponenten des Messsystems bzw. der Vorrichtung in Umgebungsobjekte des Menschen oder anderer Lebewesen integriert werden können, können einzelne Komponenten der Vorrichtung zum Erfassen am Körper des Menschen bzw. des Lebewesens angebracht werden. Dies ist dann dennoch einfacher und schneller als die Verwendung, z. B. der herkömmlichen Atmungsbänder.

### Kurzbeschreibung der Figuren

Ausführungsbeispiele der vorliegenden Erfindung werden nachfolgend unter Bezugnahme auf beiliegende Zeichnungen näher erläutert.
- Fig. 1a: zeigt eine schematische Zeichnung eines Ausführungsbeispiels einer Vorrichtung zum Erfassen einer Atmung eines Lebewesens.
- Fig. 1b: zeigt ein Ausführungsbeispiel gemäß Fig. 1a, bei der der aktive Sender und der Messfühler induktiv gekoppelt sind.
- Fig. 2: zeigt ein Blockschaltbild eines aktiven Senders eines Ausführungsbeispiels einer Vorrichtung zum Erfassen einer Atmung eines Lebewesens.
- Fig. 3: zeigt ein Blockschaltbild eines Ausführungsbeispiels eines Messfühlers bzw. einer Mess-/Empfangsvorrichtung einer Vorrichtung zum Erfassen einer Atmung eines Lebewesens.
- Fig. 4: zeigt eine schematische Darstellung eines Ausführungsbeispiels einer Vorrichtung zum Erfassen einer Atmung eines Lebewesens, die in eine Rückenlehne eines Autositzes und einen Sicherheitsgurt integriert ist, wobei das Messsignal durch eine drahtgebundene Verbindung an die Auswerteeinrichtung übermittelt wird.
- Fig. 5: zeigt oben ein Blockschaltbild eines Ausführungsbeispiels eines aktiven Senders und unten ein Blockschaltbild eines Ausführungsbeispiels eines Messfühlers für ein Ausführungsbeispiel gemäß Fig. 4.
- Fig. 6a: zeigt eine schematische Darstellung eines Ausführungsbeispiels einer Vorrichtung zum Erfassen einer Atmung eines Lebewesens, die in eine Rückenlehne eines Autositzes und einen Sicherheitsgurt integriert ist, wobei das Messsignal durch eine drahtlose Schnittstelle an die Auswerteeinrichtung übermittelt wird.
- Fig. 6b: zeigt eine schematische Darstellung eines Ausführungsbeispiels eines Sicherheitsgurtes und eines Messfühlers für eine drahtlose Übertragung der Messsignale.
- Fig. 7: zeigt oben ein Blockschaltbild eines aktiven Senders und unten ein Blockschaltbild eines Messfühlers für die Verwendung in einem Ausführungsbeispiel gemäß Fig. 6a.
- Fig. 8a: zeigt eine schematische Darstellung eines Ausführungsbeispiels einer Vorrichtung zum Erfassen einer Atmung eines Lebewesens, die in eine Rückenlehne eines Autositzes und einen Sicherheitsgurt integriert ist, wobei die drahtlose Übertragung der Messsignale mittels Lastmodulation bzw. Backscatter-Verfahren erfolgt.
- Fig. 8b: zeigt eine schematische Darstellung eines Ausführungsbeispiels eines Sicherheitsgurtes mit einem integrierten Transponder als Messfühler.
- Fig. 9: zeigt oben ein Blockschaltbild eines Ausführungsbeispiels eines aktiven Senders und unten ein Blockschaltbild eines Ausführungsbeispiels eines Messfühlers für die Verwendung in einer Vorrichtung gemäß Fig. 8a.
- Fig. 10: zeigt oben ein weiteres Ausführungsbeispiel einer Vorrichtung zum Erfassen einer Atmung eines Lebewesens, bei der der aktive Sender an einem Bett oder einer Liege angebracht ist und der Messfühler an einem Gurt, der mit dem Bett bzw. der Liege verbunden ist, und Fig. 10 zeigt unten ein weiteres Ausführungsbeispiel einer Vorrichtung zum Erfassen einer Atmung eines Lebewesens, bei der der Messfühler in einem Kissen integriert ist, das auf den Rumpf des Lebewesens aufgelegt werden kann.
- Fig. 11: zeigt eine gemessene Atmungskurve über mehrere Sekunden.
- Fig. 12: zeigt ein Ausführungsbeispiel eines Messfühlers für die induktive Kopplung.
- Fig. 13: zeigt ein Ausführungsbeispiel einer Vorrichtung zum Erfassen einer Atmung eines Lebewesens, die in die Rückenlehne eines Autositzes und in einen Sicherheitsgurt integriert ist.

Für den Begriff Vorrichtung zum Erfassen kann auch der Begriff Messsystem, für den Begriff aktiver Sender können auch die Begriffe Sendervorrichtung oder Sendereinheit verwendet werden, für den Begriff Messfühler auch die Begriffe Empfängervorrichtung oder Empfängereinheit, für die erste bzw. zweite Einrichtung auch die Begriffe erstes bzw. zweites Objekt oder Umgebungsobjekt.

### Detaillierte Beschreibung der Erfindung

Im Folgenden werden für Objekte und Funktionseinheiten, die gleiche oder ähnliche funktionelle Eigenschaften aufweisen, die gleichen Bezugszeichen verwendet, wobei auf eine wiederholte Beschreibung dieser Objekte und Funktionseinheiten verzichtet wird, um unnötige Wiederholungen zu vermeiden.

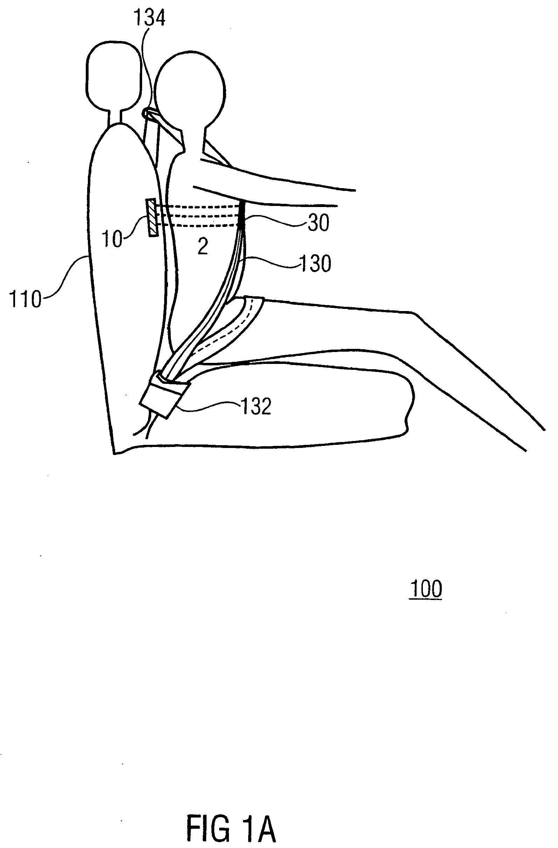

Fig. 1a zeigt eine schematische Darstellung eines Ausführungsbeispiels einer Vorrichtung 100 zum Erfassen einer Atmung eines Lebewesens, hier eines Menschen, die in eine Automobilumgebung integriert ist.

Die Vorrichtung 100 zum Erfassen weist einen aktiven Sender 10 und einen Messfühler 30 auf, wobei der aktive Sender ausgebildet ist, um ein magnetisches oder elektromagnetisches Feld zu erzeugen, und der Messfühler ausgebildet ist, um ein Signal bereitzustellen, das von dem magnetischen oder elektromagnetischen Feld und von einer durch die Atmung des Lebewesens bzw. Menschen bedingten Abstandsänderung zwischen dem aktiven Sender 10 und dem Messfühler 30 abhängt. Dabei ist der Messfühler 30 an dem Rumpf des Lebewesens angeordnet.

In dem in Fig. 1a gezeigten Ausführungsbeispiel sind der aktive Sender 10 und der Messfühler 30 auf gegenüberliegenden Seiten des Oberkörpers 2 des Menschen angeordnet.

Weitere Ausführungsbeispiele (s. Fig. 1a) weisen zusätzlich eine erste Einrichtung 110 und eine zweite Einrichtung 130 auf, wobei die erste und zweite Einrichtung derart ausgebildet und miteinander verbunden sind, dass der aktive Sender 10 derart in oder an der ersten Einrichtung 110 angeordnet ist, und der Messfühler 30 derart in oder an der zweiten Einrichtung 130 angeordnet ist, dass der aktive Sender 10 und der Messfühler 30 unabhängig von einer Drehung bzw. Drehbewegung des Rumpfs 2 des Lebewesens auf gegenüberliegenden Seiten des Rumpfs angeordnet sind bzw. bleiben.

In dem in Fig. 1a gezeigten Ausführungsbeispiel weist die erste Einrichtung eine Rückenlehne des Autositzes auf, und die zweite Einrichtung einen Sicherheitsgurt 130. Die Rückenlehne und der Sicherheitsgurt sind jedoch nur Beispiele für eine erste und zweite Einrichtung, und Ausführungsbeispiele sind nicht auf diese beschränkt. Dabei können, wie in Fig. 1a gezeigt, der aktive Sender 10 in der ersten Einrichtung 110 angeordnet bzw. integriert sein, und der Messfühler 30 an der zweiten Einrichtung 130, z. B. dem Sicherheitsgurt 130, angeordnet bzw. in dieser integriert sein, oder umgekehrt, der aktive Sender 10 in der zweiten Einrichtung 130 integriert sein und der Messfühler 30 in der ersten Einrichtung 110 integriert sein.

Dabei wird im Folgenden allgemein von "integriert" gesprochen, unabhängig davon, ob der aktive Sender 10 und/oder der Messfühler 30 ganz oder teilweise innerhalb oder außerhalb der ersten oder zweiten Einrichtung 110, 130 angeordnet sind.

In weiteren Ausführungsbeispielen ist die erste oder zweite Einrichtung 110, 130 beispielsweise ein Bett oder eine Liege, und die entsprechend andere Einrichtung ein Gurt, der mit dem Bett oder der Liege verbunden sein kann, oder beispielsweise ein Kissen, in das der aktive Sender oder der Messfühler integriert ist.

Fig. 1a zeigt ein Ausführungsbeispiel der Vorrichtung zum Erfassen der Atmung, bei dem die erste Einrichtung 110 und die zweite Einrichtung 130 miteinander verbunden sind, in Fig. 1a durch die Gurthalterung 132 und die Gurtführung 134, die wiederum fest mit der Karosserie des Fahrzeugs (nicht gezeigt) verbunden sind, und somit auch mit dem Autositz bzw. der Rückenlehne 110 des Autositzes. Die Verbindung zwischen der ersten Einrichtung 110 und der zweiten Einrichtung 130 ermöglicht, dass sich der Rumpf 2 des Menschen beispielsweise in Bezug auf seine Längsachse zwischen der ersten Einrichtung 110 und der zweiten Einrichtung 130 drehen kann, und der aktive Sender 10 und der Messfühler 30 dennoch auf gegenüberliegenden Seiten des Rumpfs 2 angeordnet sind, und somit eine Messung der Abstandsänderung zwischen dem aktiven Sender 10 und dem Messfühler 30 als Maß für die Atmungsanstrengung ermöglichen. Um diese Bewegung zu ermöglichen, kann eine der beiden Einrichtungen oder beide elastisch sein oder elastisch befestigt sein, siehe z. B. der Sicherheitsgurt mit der elastischen Aufrollmechanik.

In weiteren Ausführungsbeispielen ist ein Sicherungsgurt als zweite Einrichtung 130 mit dem Bett oder der Liege als erste Einrichtung 110 verbunden, um trotz einer Drehung des Rumpfes eine durch die Atmung verursachte Abstandsänderung zuverlässig zu messen.

Obwohl das Ausführungsbeispiel in Fig. 1a eine Vorrichtung mit einer ersten und zweiten Einrichtung, die miteinander verbunden sind, zeigt, können alternative Ausführungsbeispiele eine erste und zweite Einrichtung aufweisen, die nicht miteinander verbunden sind oder keine erste oder zweite Vorrichtung aufweisen.

Allgemein kann daher gesagt werden, dass die Vorrichtung zum Erfassen bzw. zur Messung der Atmungsanstrengung einen aktiven Sender 10, der auch als Sendervorrichtung bezeichnet wird, und einen Messfühler, der im Folgenden auch als Empfängervorrichtung 30 bezeichnet wird, aufweist, wobei die Messung auf einer Abstandsänderung zwischen Sendereinheit und Empfängereinheit beruht und somit messtechnisch erfasst werden kann. Die Messsignale im Empfänger können dabei durch unterschiedliche physikalische Beeinflussungen zwischen der Sendereinheit und der Empfängereinheit hervorgerufen werden, z. B. durch magnetische Felder oder elektromagnetische Felder.

Im Folgenden werden Ausführungsbeispiele näher beschrieben, die auf der aktiven Erzeugung eines magnetischen Felds durch die Sendervorrichtung 10 basieren, d. h. auf einer induktiven Kopplung basieren. In derartigen Ausführungsbeispielen besteht die Vorrichtung 100, die im Folgenden auch allgemein als Messsystem bezeichnet wird, im Wesentlichen aus zwei miteinander lose induktiv gekoppelten Spulen. Die Primärspule kann dabei in ein Objekt, z. B. einen Autositz oder einen Operationstisch, integriert werden und erzeugt ein magnetisches Wechselfeld mit einer festgelegten Frequenz, Amplitude und/oder Phasenlage. Zur Stabilisierung bzw. zur Messung von Störeinflüssen oder zur Messung von Amplitudenschwankungen kann die Sendeeinheit und gegebenenfalls auch die Empfangsvorrichtung zusätzliche Messspulen aufweisen bzw. können zusätzliche Messspulen im Umgebungsobjekt integriert werden.

Zur Messung der Atmungsanstrengung wird eine Sekundärspule bzw. ein Sekundärspulensystem als Messfühler in einem bestimmten Abstand von der Primärspule bzw. dem Primärspulensystem des aktiven Senders 10 an dem zu messenden Objekt in der Art befestigt, dass eine Bewegungsänderung zwischen den beiden Spulen bzw. Spulensystemen sich in Form einer Messgröße widerspiegelt. Bei Ausführungsbeispielen, die auf der induktiven Kopplung basieren, handelt es sich bei der zu ermittelnden Messgröße bzw. dem zu erzeugenden Signal um eine Änderung der in der Sekundärspule erzeugten Induktionsspannung, die aufgrund der Ortsabweichung der Sekundärspule in Bezug auf die Primärspule entsteht. Dabei wird nicht die Induktionsspannung selbst, sondern die Änderung der Induktionsspannung, die aufgrund der Ortsabweichung von einer statischen Position oder Ausgangsposition entsteht, erfasst und ausgewertet.

Die Datenübermittlung des derart erzeugten Messsignals kann dabei auf mehreren Wegen erfolgen: a) die Messeinheit bzw. der Messfühler 30 kann drahtgebunden an die Auswerteeinheit angebunden werden, b) der Messfühler 30 kann drahtlos, z. B. über Zigbee oder Bluetooth, an die Auswerteeinheit (in Fig. 1a nicht gezeigt) angebunden werden, und c) die Messeinheit 30 kann ihre Daten über eine Rückkopplung der Sekundärspule 30 auf die Primärspule 10 mittels Lastmodulation übertragen, so dass die Datensignale dort detektiert werden können.

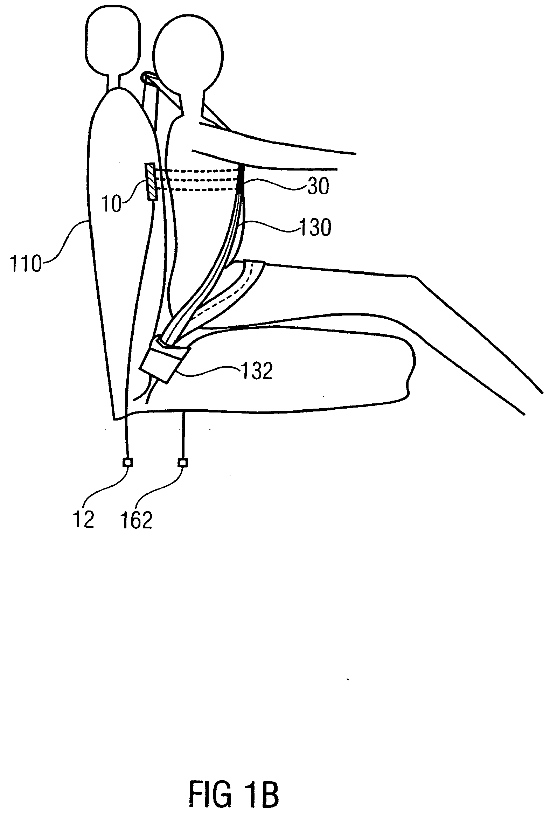

Fig. 1b zeigt ein Ausführungsbeispiel einer Vorrichtung zum Erfassen einer Atmung eines Menschen, in der die Atmungsfrequenz der im Automobil befindlichen Person überwacht werden soll. Der aktive Sender 10 und der Messfühler 30 sind induktiv gekoppelt. Der aktive Sender 10 weist eine Primärspule auf und gegebenenfalls weitere Referenzspulen, die in der Rückenlehne des Autositzes 110 integriert sind. Der aktive Sender 10 ist ausgebildet, um das magnetische Feld mittels einer in die Primärspule eingespeisten Stroms zu erzeugen. Der Messfühler 30 weist eine Sekundärspule auf und ist in den Sicherheitsgurt 130 integriert. Der Sicherheitsgurt 130 liegt, z. B. während der Fahrt bzw. im angeschnallten Zustand, eng am Brustkorb des Fahrers an. Über die induktive Kopplung durchsetzt der von der Primärspule erzeugte magnetische Fluss die Sekundärspule und induziert dabei eine Spannung, die durch einen Schwingkreis mit einem Parallelkondensator noch erhöht werden kann. Durch eine anschließende Demodulationsschaltung wird nicht die Induktionsspannung selbst ermittelt, sondern eine Änderung der Induktionsspannung. Somit kann die Atmungsaktivität des Autofahrers überwacht werden. Atmet der Autofahrer nicht, so erzeugt die Messschaltung kein Messsignal bzw. lediglich ein "Nullsignal".

Im Gegensatz zu herkömmlichen Atemmesssystemen, die wie zuvor dargelegt, ein Anlegen von sogenannten "Anwendungsteilen" (z. B. Atmungsbändern) an den Fahrer ist nicht notwendig. Nach dem Einsteigen in das Fahrzeug und dem Anschnallen des Sicherheitsgurts kann die Messung der Atmung sofort beginnen. Damit ist auch die Atmungsüberwachung wechselnder Autofahrer ohne Probleme möglich. Die Systemkomponenten und die Zuleitungen können dabei so ausgelegt werden, dass sie von außen unauffällig oder sogar nicht erkennbar sind.

Das Erfassen bzw. die Messung der Signale, die ein Maß für die Atmungsaktivität des Fahrers sind, sind während der Fahrt möglich, so dass eine Bewertung des Zustandes des Fahrzeugführers anhand der aus dem Messsignal bzw. Rohsignal abgeleiteten Größen auch während der Fahrt möglich ist.

In dem in Fig. 1b gezeigten Ausführungsbeispiel der Vorrichtung zum Erfassen der Atmung erfolgt die Anbindung der Auswerteeinheit drahtgebunden, siehe Bezugszeichen 162 für den Anschluss zum Abgreifen des Sensorsignals bzw. Messsignals von dem Messfühler 30. Bezugszeichen 12 bezeichnet den Anschluss für die Stromversorgung des aktiven Senders 10. Alternative Ausführungsbeispiele übertragen die Daten von dem Messfühler an die Auswerteeinheit per Funk, Lastmodulation, Backscatter-Verfahren oder ähnlichen Verfahren.

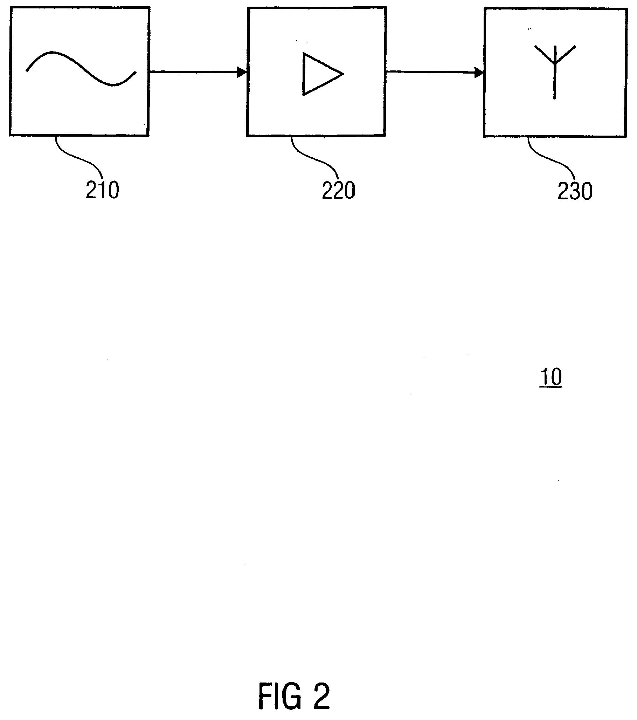

Fig. 2 zeigt ein Blockdiagramm eines Ausführungsbeispiels eines aktiven Senders 10, der einen Signalgenerator 210, einen Verstärker 220 und eine Antenneneinheit 230 aufweist. Der Signalgenerator 210 ist ausgebildet, über ein an den Verstärker 220, z. B. ein Leistungsverstärker oder eine Endstufe, angelegtes Signal (siehe Pfeil) einen Strom in die primäre Antenneneinheit (siehe Pfeil), die eine Primärspule und ein Anpassungsnetzwerk aufweist, einzuspeisen und somit ein magnetisches Wechselfeld mit einer festgelegten Frequenz, Amplitude und gegebenenfalls einer definierten Phasenlage zu erzeugen. Der Signalgenerator 210 ist beispielsweise ein Sinus-Oszillator.

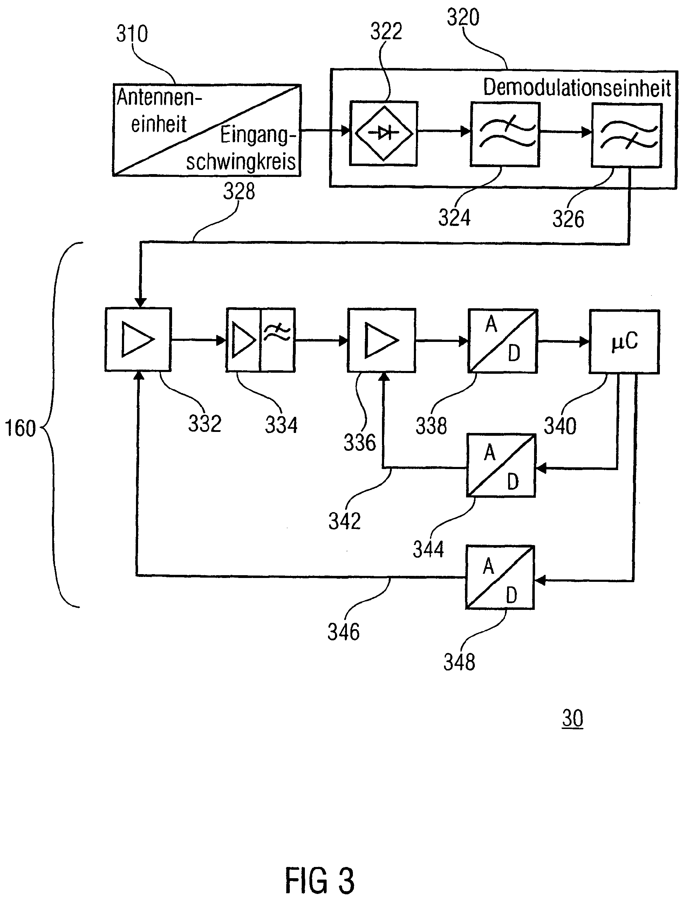

Fig. 3 zeigt ein Blockdiagramm eines Ausführungsbeispiels eines Messfühlers bzw. einer Messvorrichtung 30 mit einer Antenneneinheit 310, einer Demodulationseinheit 320 und einer Signalverarbeitungseinheit 160. Das sinusförmige Signal der Primärspule 230 bzw. des aktiven Senders 10 aus Fig. 2 induziert in die Antenneneinheit 310 der Sekundärseite bzw. des Messfühlers 30 eine sinusförmige Wechselspannung. Die Antenneneinheit 310 weist beispielsweise einen Parallelschwingkreis inklusive eines Anpassungsnetzwerks auf. Der Parallelschwingkreis, der die Sekundärspule und einen Parallelkondensator aufweist, dient hier für die induktive Kopplung zwischen der Sekundärspule und der Primärspule des aktiven Senders, sowie einer Erhöhung der induzierten Spannung. Durch die nachfolgende Demodulationseinheit 320 wird die Spannungsänderung detektiert. In einem einfachen Fall besteht die Demodulationseinheit 320 aus einem Gleichrichter 322, der zwei verschiedenen Filterstufen 324 und 326 vorgeschaltet ist. Dabei kann beispielsweise die erste Filterstufe ein Tiefpassfilter sein und die zweite Filterstufe ein Hochpassfilter bzw. Kopplungskondensator sein (siehe Fig. 3). Der Gleichrichter 322 invertiert dabei negative Signalanteile der induzierten Spannung. Das durch die anschließende Filterung (Tiefpassfilterung 324 und Abtrennung des Gleichanteils 326) empfangene Signal 328 am Ausgang der Demodulationseinheit enthält bereits das Atemsignal.

In Fig. 3 sind weitere Verstärker-, Filter-, Anpass- und Wandlerstufen zur weiteren Signalverarbeitung des "Atemsignals" 328 gezeigt, sowie eine Einheit zur Steuerung und Auswertung der Signale, hier einem Mikrocontroller µC. Dabei bezeichnet Bezugszeichen 332 einen Impedanzwandler, Bezugszeichen 334 einen Verstärker mit einem aktiven Filter, Bezugszeichen 336 einen Verstärker mit "Gain-Regelung", Bezugszeichen 338 einen Analog/Digital-Wandler (AD-Wandler) und 340 den Mikrocontroller. Die erste Rückkopplung 342 von dem Mikrocontroller 340 auf den Verstärker mit Gain-Regelung 336 dient der Verstärkungsregelung (Gain), während die zweite Rückkopplung 346 von dem Mikrocontroller 340 auf den Impedanzwandler 332 einer dynamischen Offset-Korrektur dient. Sowohl die erste Rückkopplung 342 als auch die zweite Rückkopplung 346 weisen jeweils einen AD-Wandler 344 bzw. 348 auf. In weiteren Ausführungsbeispielen kann implizit vor der Analog/Digital-Wandlung 338 ein Anti-Aliasing-Filter, sowie nach den Digital/Analog-Wandlungen 344, 348 ein Rekonstruktionsfilter verwendet werden, was aus Übersichtsgründen nicht in der Figur dargestellt ist.

Die zuvor beschriebenen Ausführungsbeispiele bezogen sich auf eine Signaländerungen bzw. Änderungen des Signals 328, die durch eine Abstandsänderung zwischen einem aktiven Sender 10 und einem Messfühler 30, die induktiv miteinander gekoppelt sind, hervorgerufen werden. Alternativ können Ausführungsbeispiele statt der induktiven Kopplung zwischen dem aktiven Sender 10 und dem Messfühler 30 auch andere Kopplungsmechanismen aufweisen, z. B. eine elektromagnetische Kopplung.

Der aktive Sender 10 bzw. die Sendereinheit 10 eines Ausführungsbeispiels mit elektromagnetischer Kopplung weist beispielsweise eine Dipol- oder Patch-Antenne auf, die durch ein hochfrequentes Signal gespeist wird. Die Signalerzeugung selbst ist dabei ähnlich der der induktiv gekoppelten Systeme bzw. Ausführungsbeispiele. Ähnliches gilt für Ausführungsbeispiele der Empfangsantenne, aus deren Empfangssignaländerung, die durch eine Feldstärkeänderung hervorgerufen wird, die Atmungsanstrengung abgeleitet wird. Ähnlich dem aktiven Sender 10 weist der Messfühler 30 z. B. Dipol- oder Patch-Antennen auf.

Das detektierte Empfangssignal wird anschließend in ähnlicher Weise wie bei induktiv gekoppelten Ausführungsbeispielen weiter verarbeitet und ausgewertet. Im Falle von Ausführungsbeispielen mit einem Transponder als Messfühler wird anstelle der Lastmodulation ein Backscatter-Verfahren verwendet, um das Messsignal an die Auswerteeinheit zu übertragen. Ausführungsbeispiele mit einem Transponder als Messfühler haben den Vorteil, dass diese für den Betrieb keine zusätzliche Stromversorgung neben der für den aktiven Sender benötigen, sondern die Stromversorgung über die induktive bzw. elektromagnetische Kopplung erfolgt.

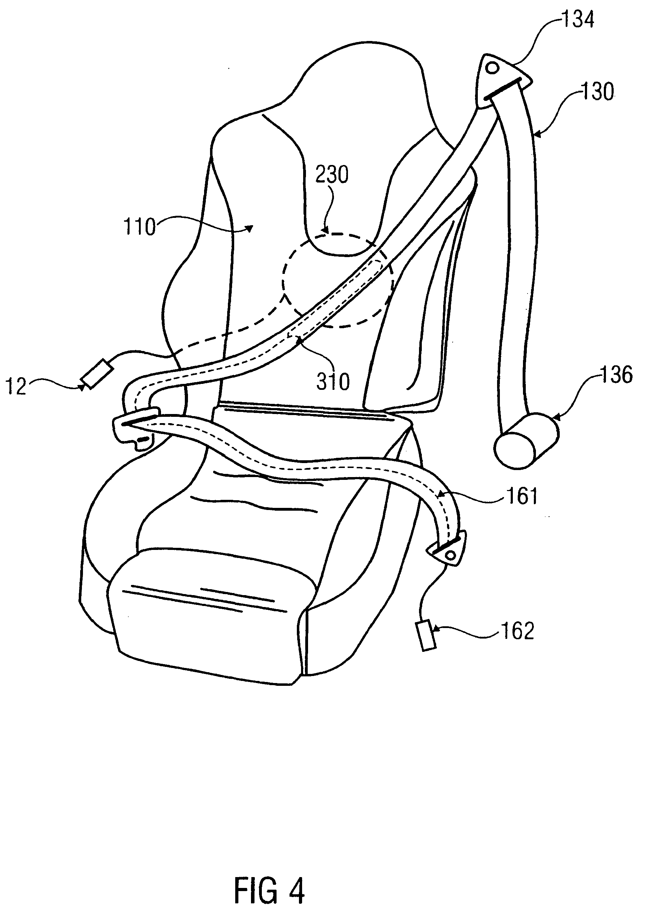

Fig. 4 zeigt eine schematische Darstellung eines Ausführungsbeispiels einer Vorrichtung zum Erfassen bzw. Überwachen einer Atmungsfrequenz einer im Automobil befindlichen Person, wobei die Anbindung der Messeinheit an die Auswerteeinheit über eine verdrahtete Anbindung erfolgt.

Die Vorrichtung zum Erfassen der Atmung einer Person gemäß Fig. 4 weist einen Autositz bzw. die Rückenlehne eines Autositzes als erste Einrichtung 110 auf, einen Sicherheitsgurt als zweite Einrichtung 130, einen aktiven Sender 10 und einen Messfühler 30 auf. Dabei ist in Fig. 4 genauer gesagt die Primärspule 230 des aktiven Senders 10 (siehe gestrichelte Linie) und die Sekundärspule 310 des Messfühlers 30 (siehe gestrichelte Spule im Gurtbereich) gezeigt.

Die erste Einrichtung 110 ist über die Karosserie mit der zweiten Einrichtung 130 verbunden, die z. B. über die Gurtführung 134 und den Gurtretraktor 136 mit derselben Karosserie verbunden ist.

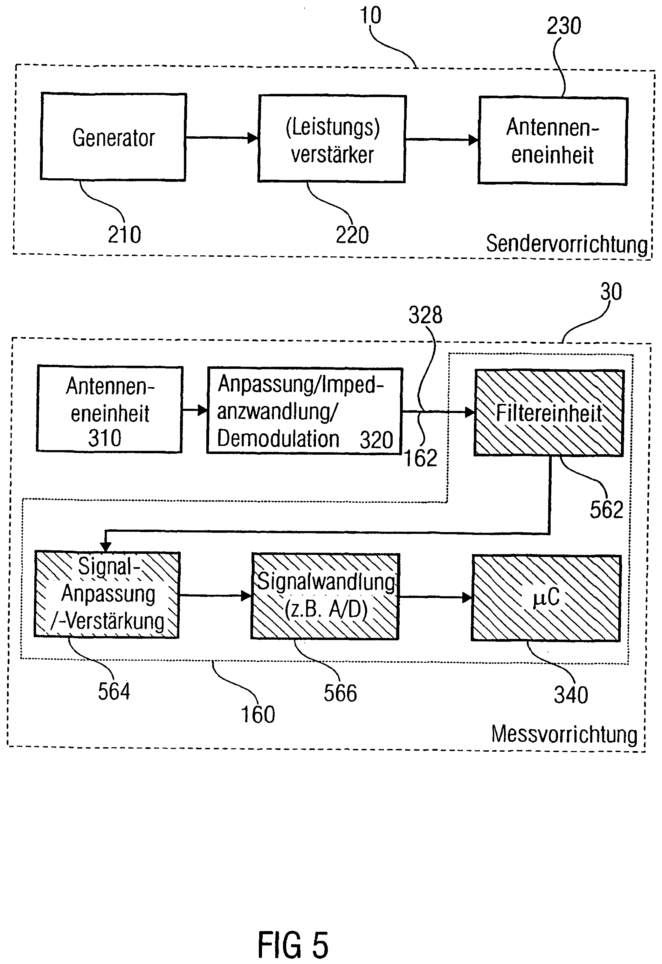

Fig. 4 zeigt ein Ausführungsbeispiel einer Integration einer Vorrichtung zum Messen der Atmungsanstrengung in Automobilumgebungen. Der aktive Sender bzw. die Primärspule 230 ist in der Rückenlehne 110 des Automobilsitzes gegenüber dem Brustkorb des Fahrers integriert. Durch den Anschluss 12 wird ein von außen erzeugtes und verstärktes sinusförmiges Signal, z. B. ein sinusförmiger Strom oder eine sinusförmige Spannung, in die Primärspule 230 eingespeist und somit an der Antenne, d. h. der Primärspule 230, ein magnetisches Feld erzeugt. Weitere Details des aktiven Senders werden später anhand von Fig. 5 oben näher erläutert. Alternativ kann die komplette Sendervorrichtung 10 bestehend aus Signalerzeugung 210, Signalverstärkung 220 und der Sendeantenne 230 direkt in die Rückenlehne 110 des Automobilsitzes integriert werden. In diesem Fall gibt es keinen Anschluss 12 nach außen.

Der Messfühler 30 bzw. die Empfangsvorrichtung 30 weist eine Sekundärspule 310 sowie eine Anpassungs-, Impedanzwandlungs- und Demodulationselektronik 320 auf. Die Windungen der Sekundärspule 310 sind beispielsweise in den Sicherheitsgurt 130 eingewebt oder auf einer Trägerfolie eingebracht und abschließend in den Sicherheitsgurt eingeklebt bzw. eingeschweißt. Die Anpassungs-, Impedanzwandlungs- und Demodulationselektronik ist vorzugsweise in unmittelbarer Nähe von der Sekundärspule 310 in dem Sicherheitsgurt 130 integriert, z. B. in Form einer kleinen Plastikkapsel (nicht in der Zeichnung dargestellt), die in den Sicherheitsgurt eingeschweißt ist. Die Anbindung der Empfangsvorrichtung 30 an die Auswerteeinheit (nicht gezeigt) erfolgt über eine Leitung 161 (siehe gestrichelte Linie in dem Gurt), die entlang des Gurtes 130 geführt ist. Die Leitung 161 kann beispielsweise direkt in den Sicherheitsgurt eingewebt sein oder in Form von flachen und dünnen Drähten entlang des Sicherheitsgurtes befestigt sein. Diese Leitung 161 verläuft ausgehend von der Empfangsvorrichtung 30 oder, in anderen Worten, von der Sekundärspule 310 mit der Anpassungs-, Impedanzwandlungs- und Demodulationselektronik 320, bis hin zu der Gurtverankerung auf der gegenüberliegenden Seite. Alternativ kann die Leitung 161 in die andere Richtung entlang des Sicherheitsgurts bis hin zu dem Gurtretraktor 136 geführt werden. Beide Varianten sind technisch möglich. Die erste Variante kann jedoch technisch einfacher implementiert werden. Die Leitung 161 ist mit dem Anschluss 162 zum Abgreifen des Sensorsignals terminiert.

Ein Ausführungsbeispiel einer Messvorrichtung 30 ist unten in Fig. 5 schematisch dargestellt. Die Messvorrichtung bzw. der Messfühler 30 weisen die Antenneneinheit 310, die Anpassungs-, Impedanzwandlungs- und Demodulationseinheit 320 und die Signalverarbeitungseinheit 160 auf (grau schattierte Systemeinheiten in Fig. 5, umrandet von punktierter Linie). Die Funktionsblöcke bzw. Systemeinheiten der Signalverarbeitungseinheit 160 sind gemäß einem Ausführungsbeispiel nicht in dem Sicherheitsgurt 130 integriert, sondern in einem externen Gehäuse untergebracht, das an den Anschluss 162 angeschlossen ist. Die Signalverarbeitungseinheit 160 weist, wie z. B. anhand von Fig. 3 schon erläutert, eine Filtereinheit 562, eine Signal-Anpassungs/Verstärkungs-Einheit 564, eine Signalwandlungsz. B. Analog/Digital-Wandlungseinheit 566 und einen Mikrocontroller 340 auf.

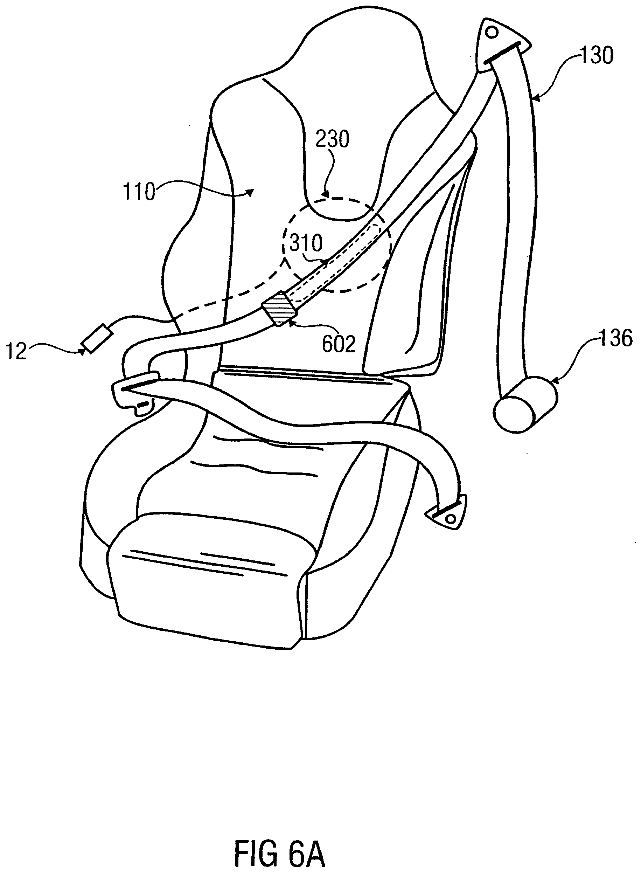

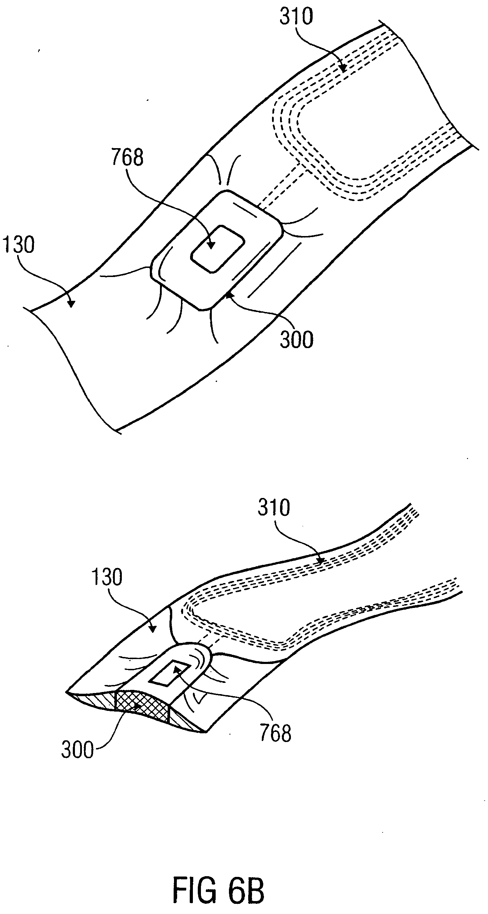

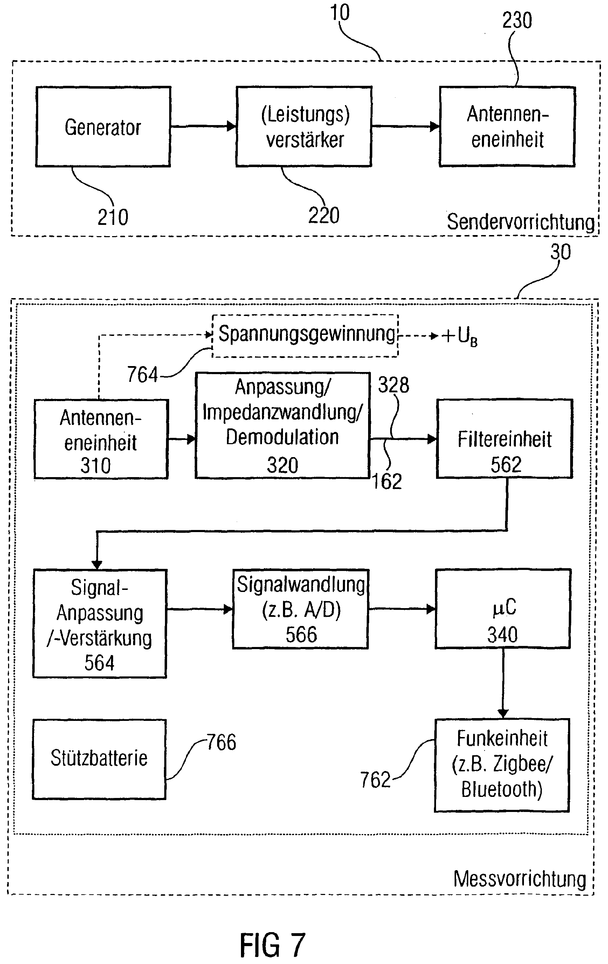

Fig. 6a zeigt ein weiteres Ausführungsbeispiel einer Vorrichtung zum Überwachen der Atmungsfrequenz einer im Automobil befindlichen Person, wobei die Messeinheit 30 mittels einer drahtlosen Anbindung an die Auswerteeinheit gekoppelt ist. Dabei zeigen die Fig. 6a, 6b ein Ausführungsbeispiel für eine Integration des Systems zur Messung der Atmungsanstrengung in einer Automobilumgebung ähnlich, wie sie in Fig. 4 gezeigt wurde. Die Ausführung der Sendervorrichtung bzw. des aktiven Senders 10 (s. Fig. 7 oben) kann dabei die gleiche sein wie in Fig. 4, d. h. unterscheidet sich nicht von der in Fig. 4.

Die Empfangs- oder Messvorrichtung 30 weist in diesem Ausführungsbeispiel zusätzlich die Funktionen zur Datenverarbeitung und drahtlosen Datenübermittlung auf und kann, z. B., in Form eines elektronischen Datenverarbeitungsmoduls in einem kleinen Gehäuse 602 direkt auf dem Sicherheitsgurt 130 befestigt werden (s. Fig. 6a). Die Komponenten bzw. Funktionselemente der Messvorrichtung 30 sind unten in Fig. 7 dargestellt. Im Vergleich zu der Messvorrichtung in Fig. 5 weist die Messvorrichtung in Fig. 7 zusätzlich eine Funkeinheit 762, ein Spannungsgewinnungsmodul 764 und gegebenenfalls eine Stützbatterie 766 auf. Mittels dieser Einheiten werden die Atmungsrohsignale 328 direkt im Sicherheitsgurt erfasst und per Funk an die Auswerteeinheit übertragen.

In diesen Ausführungsbeispielen ist daher keine weitere Leitung von der Empfangsvorrichtung 300 bis hin zu der Gurtverankerung bzw. dem Gurtretraktor notwendig. Das Spannungsgewinnungsmodul 764 kann ausgewählt sein, die gesamte Elektronikschaltung der Empfangsvorrichtung 300 mit Strom zu versorgen. Für den Fall, dass der Stromverbrauch der Funkeinheit 762 die Kapazität des Spannungsgewinnungsmoduls 764 überschreitet, kann optional eine Stützbatterie 766 vorgesehen werden, die über einen Batteriefachdeckel 768 jederzeit gewechselt werden kann. Dabei kann die Stützbatterie mit einer integrierten Ladeschaltung im laufenden Betrieb auch aus dem umgebenden Feld geladen werden, z.B. durch einen speziellen Modus, der immer dann aktiv ist, wenn keine Messungen erfolgen, und somit die Elektronik weniger Energie benötigt.

Fig. 7 zeigt oben das Blockdiagramm des aktiven Senders 10, wie es schon basierend auf Fig. 5 beschrieben wurde.

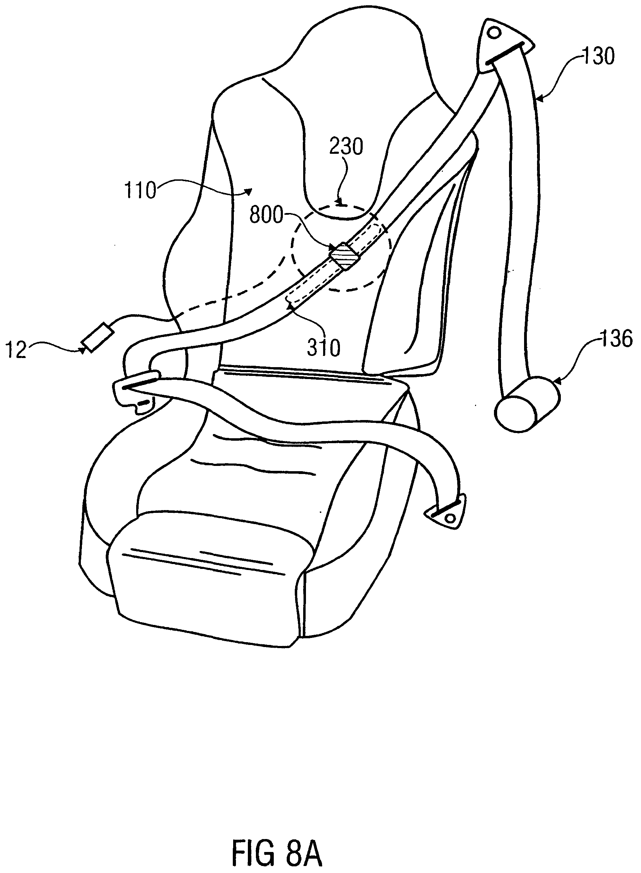

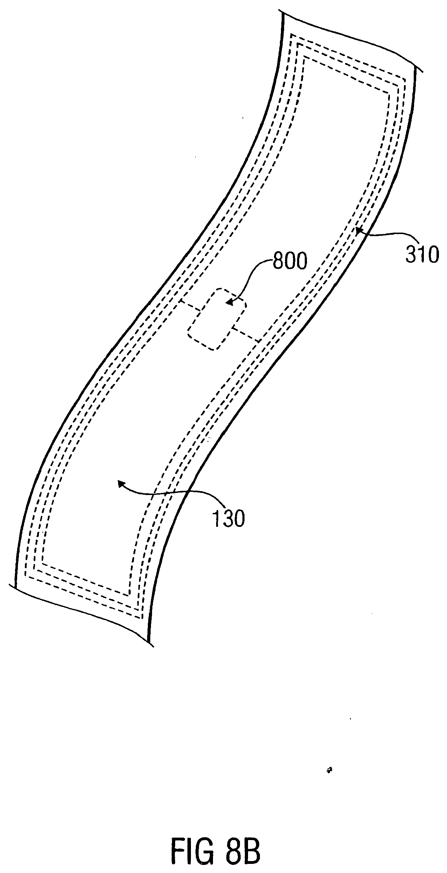

Im Folgenden wird ein weiteres Ausführungsbeispiel einer Vorrichtung zur Überwachung der Atmungsfrequenz einer im Automobil befindlichen Person beschrieben, bei der die Datenübertragung mittels einer Rückkopplung der Sekundärspule auf die Primärspule durch Lastmodulation erfolgt. Die Fig. 8a und 8b zeigen ein Ausführungsbeispiel einer Integration des Systems zur Messung der Atmungsanstrengung in einer Automobilumgebung.

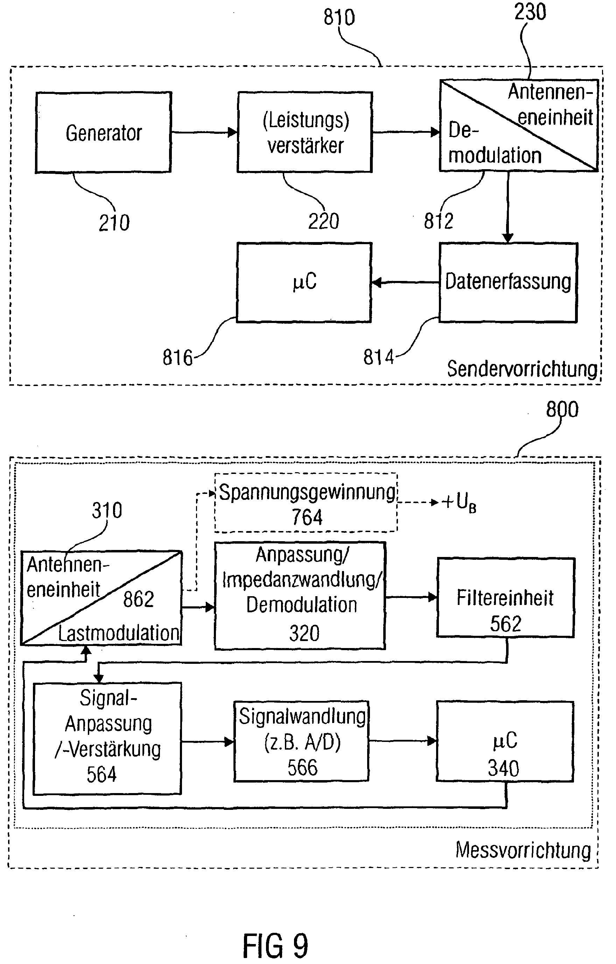

Die Sendervorrichtung bzw. der aktive Sender 810, siehe Fig. 9 oben, unterscheidet sich von der Sendervorrichtung 10 aus den Fig. 5 und 7 dadurch, dass die Sendervorrichtung 810 zusätzlich eine Demodulationseinheit 812, eine Datenerfassungseinheit 814 und einen Mikrocontroller 816 aufweist. Diese zusätzlichen Funktionseinheiten dienen der Datenübertragung von der Messeinrichtung 800 an die Sendervorrichtung 810 mittels Lastmodulation.

Die Empfangsvorrichtung bzw. Messvorrichtung 800, siehe Fig. 9 unten, ist im Sicherheitsgurt 130 integriert und besteht aus der Antenneneinheit 310 bzw. Sekundärspule 310 und der Signalverarbeitungseinrichtung 160. Die Windungen der Sekundärspule 310 sind in dem Sicherheitsgurt eingewebt und die Messeinheit 800 ist beispielsweise in einer kleinen Plastikkapsel in den Sicherheitsgurt eingeschweißt bzw. an dem Sicherheitsgurt angeklebt oder eingeklebt, so dass sie von außen unauffällig bis nicht erkennbar sind. Die Messeinheit 800 wird dabei durch die aus dem Feld gewonnene Induktionsspannung bzw. den Induktionsstrom versorgt. Die Messgröße, d. h. die Änderungen der in der Sekundärspule erzeugten Induktionsspannungen, entsteht durch Ortsabweichungen der Sekundärspule 310 von der Primärspule 230. Sie korreliert direkt mit den Atmungsbewegungen am Brustkorb. Diese Änderungen der Induktionsspannungen werden durch die Elektronik detektiert, angepasst und in ein digitales Signal umgewandelt. Dieses digitale Signal wird mittels des Lastmodulationsprinzips an die Primärspule 230 übertragen, d. h. das digitalisierte Atmungsanstrengungssignal wird durch Variation der Kopplung der Sekundärspule 310 mit der Primärspule 230 an die Sendervorrichtung 810 übertragen. Dazu weist die Messvorrichtung 800 eine Lastmodulationseinheit 862 als Funkeinheit 762 gemäß Fig. 7 auf. Die Daten werden anschließend in der Sendervorrichtung 810 weiter verarbeitet bzw. durch den Anschluss 12 an die Auswerteeinheit weitergegeben.

Im Folgenden werden weitere Ausführungsbeispiele der Vorrichtung zum Erfassen der Atmung, z. B. zum Überwachen der Atmungsfrequenz, einer in einem Funktionsbett bzw. auf einem Operationstisch befindlichen Person.

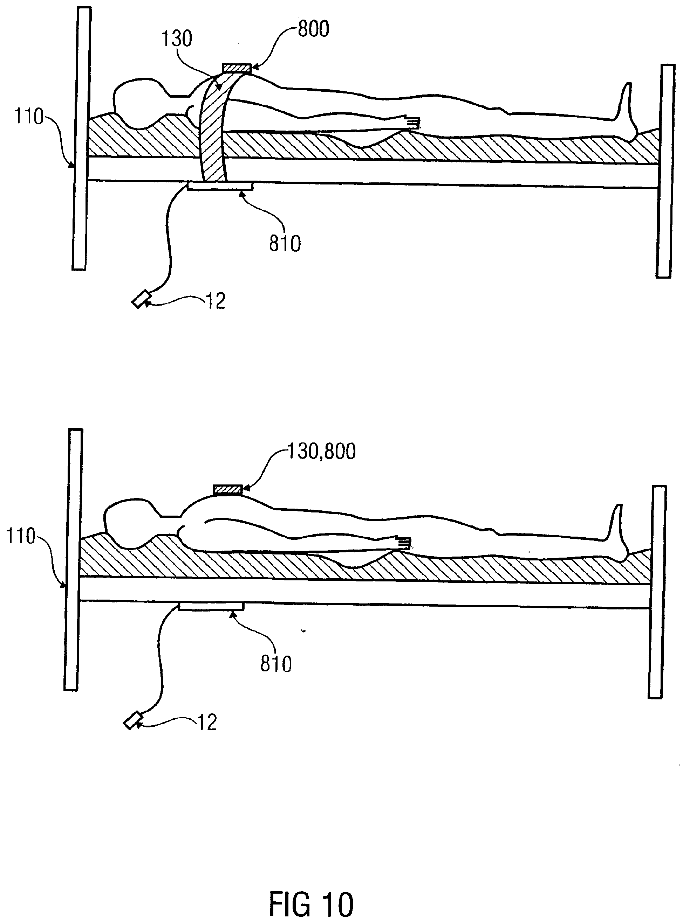

Fig. 10 oben zeigt ein Funktionsbett oder einen Operationstisch oder ähnliches als erste Einrichtung 110, einen Patientenfixiergurt als zweite Einrichtung 130, den aktiven Sender 810, der auf der Unterseite des Funktionsbetts 110 angeordnet ist, und den Messfühler bzw. der Messvorrichtung 800, die auf oder in den Gurt 130 integriert ist, so dass der aktive Sender 810 und der Messfühler 800 auf gegenüberliegenden Seiten des Rumpfs des Lebewesens, hier eines Menschen, angeordnet sind.

Das in Fig. 10 unten dargestellte Ausführungsbeispiel unterscheidet sich durch das in Fig. 10 oben dargestellte dadurch, dass der Messfühler 800 nicht in einem Patientenfixiergurt 130 integriert ist, sondern in beispielsweise einem Kissen, das während der Operation auf den Brustkorb des Patienten gelegt werden kann, um die Atmung desselben zu überwachen. In beiden Ausführungsbeispielen gemäß Fig. 10 wird die Signalübertragung mittels Lastmodulation, wie anhand von Fig. 8a, 8b und 9 beschrieben, verwendet.

Es sei jedoch darauf hingewiesen, dass auch andere Datenübertragungstechniken, wie z. B. andere drahtlose oder drahtgebundene Übertragungstechnologien, wie sie ebenfalls beispielhaft zuvor dargestellt wurden, eingesetzt werden können.

In anderen Worten, Ausführungsbeispiele gemäß Fig. 10 unterscheiden sich von den zuvor beschriebenen Ausführungsbeispielen für die Integration in einem Automobil lediglich dadurch, dass hier andere umgebungsbezogene Integrationsobjekte bzw. erste und zweite Einrichtungen 110, 130 eingesetzt werden. Das Messprinzip bleibt gleich. Hier wird die Sendereinheit mit der Primärspule 230 unter dem Funktionsbett bzw. Operationstisch 110 gegenüber dem Brustkorb des Patienten befestigt und die Messeinheit 800 mit der Sekundärspule 310 und der Lastmodulation auf dem Brustkorb des Patienten angelegt. Dabei gibt es, wie zuvor dargelegt, mehrere Anwendungsvarianten, wie man die Messeinheit 800 auf dem Brustkorb des Patienten befestigt. Sie kann beispielsweise in den Patientenfixiergurt 130 integriert werden (siehe Fig. 10 oben) oder in Form eines Gummikissens oder ähnlichem (siehe Fig. 10 unten) einfach auf den Brustkorb aufgelegt sein.

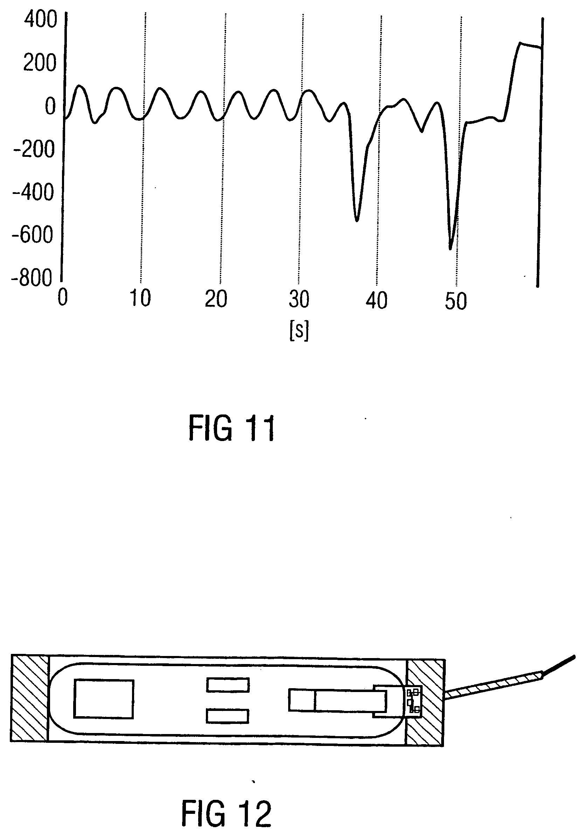

Fig. 11 zeigt ein beispielhaftes Diagramm einer Atmungskurve über mehrere Sekunden. Auf der x-Achse ist die Zeit in Sekunden aufgetragen und auf der y-Achse ein Maß für die positive und negative Änderung des Abstands zwischen dem aktiven Sender und dem Messfühler bzw. der negativen und positiven Abweichung von einem Bezugsabstand zwischen dem aktiven Sender und dem Messfühler.

Fig. 12 zeigt ein Beispiel eines Prototypen der Empfängereinheit mit Sekundärspule bzw. dem Messfühler, der eine integrierte Anpassungs- und Demodulationselektronik aufweist.

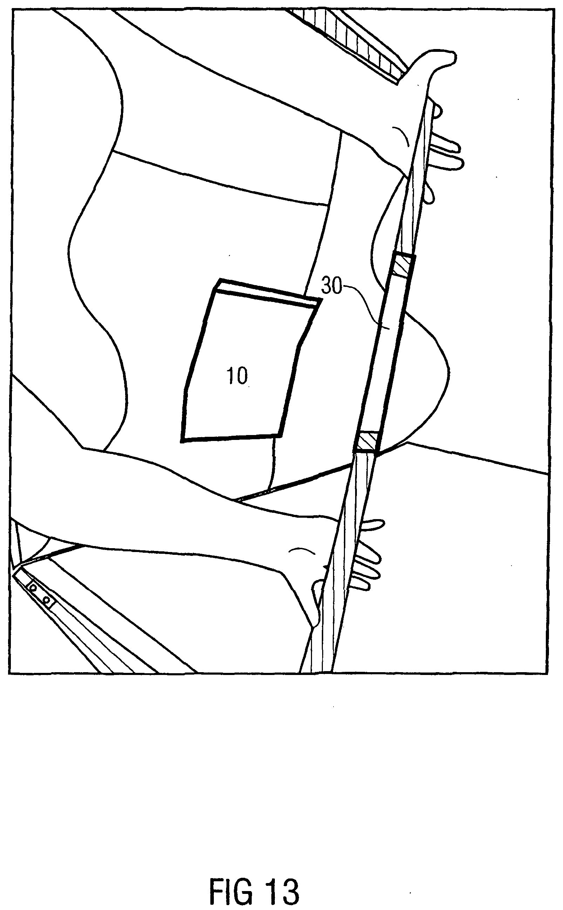

Fig. 13 zeigt ein Ausführungsbeispiel der Vorrichtung zum Erfassen einer Atmung eines Lebewesens, wobei der aktive Sender 10 in der Rückenlehne des Autositzes integriert ist (siehe weiße Umrandung) und der Messfühler bzw. die Empfängereinheit 30 in dem Rückhaltesystem bzw. Sicherheitsgurt integriert ist.

Zusammenfassend kann daher gesagt werden, dass Ausführungsbeispiele der vorliegenden Erfindung eine "Vorrichtung und Verfahren zur Messung der Atmungsanstrengung", weitere Ausführungsbeispiele eine "Vorrichtung und Verfahren zur Messung der Atmungsanstrengung mittels lose induktiv gekoppelter Spulen" und wiederum weitere Ausführungsbeispiele eine "Vorrichtung und Verfahren zur Messung der Atmungsanstrengung, Atmungsfrequenz, Atmungsamplitude und Atmungsvolumen mittels lose induktiv gekoppelter Spulen" realisieren.

Ausführungsbeispiele der vorliegenden Erfindung betreffen zudem ein Verfahren und eine Vorrichtung zur Messung der Atmungsanstrengung, die eine Sendervorrichtung und eine Empfangsvorrichtung aufweisen, wobei die Empfangsvorrichtung auf eine Abstandsänderung zwischen Sendereinheit und Empfängereinheit beruht und somit messtechnisch erfasst werden kann. Die Messsignale im Empfänger können dabei durch unterschiedliche physikalische Beeinflussungen zwischen Sendereinheit und Empfängereinheit hervorgerufen werden.

Varianten dieser Ausführungsbeispiele umfassen ferner eine Vorrichtung und ein Verfahren zur Erfassung der Atmungsbewegungen am Rumpf eines Lebewesens mittels induktiv gekoppelter Spulen, wobei Atmungsbewegungen sich in Form von Ortsabweichungen der Sekundärspule gegenüber der Primärspule insofern erkennbar machen, dass sie Änderungen der induzierten Spannung in der Sekundärspule verursachen.

Weitere der oben genannten Ausführungsbeispiele schaffen eine Vorrichtung und ein Verfahren zum Erfassen der Atmungsanstrengung, der Atmungsfrequenz, der Atmungsamplitude und des Atmungsvolumens anhand der Atmungsbewegungen, die am Rumpf eines Lebewesens in Form von Ortsabweichungen der Sekundärspule von der Primärspule erkennbar sind.

Weitere Varianten der zuvor genannten Ausführungsbeispiele beziehen sich auf eine Messvorrichtung zum Erfassen der Atmungsbewegungen am Rumpf eines Lebewesens mittels induktiv gekoppelter Spulen.

Wiederum weitere Varianten der zuvor genannten Ausführungsbeispiele der Vorrichtung stellen eine Messvorrichtung zum Erfassen der Atmungsbewegungen am Rumpf eines Lebewesens mittels induktiv gekoppelter Spulen (Primärspule und Sekundärspule) bereit, wobei das Messsystem zur Stabilisierung bzw. zur Messung von Störeinflüssen oder zur Messung von Amplitudenschwankungen mit zusätzlichen Messspulen ausgestattet ist, oder in anderen Worten, wobei das Primärspulensystem und das Sekundärspulensystem zusätzliche Messspulen aufweisen.

Weitere Ausführungsbeispiele der Vorrichtung stellen ein in den Sitz und den Sicherheitsgurt integriertes Messsystem zum Erfassen der Atmungsanstrengung als kontinuierliches Signal bereit. Dabei kann die Atmungsanstrengung an mindestens einer, aber auch an mehreren Körperpartien gemessen werden. Dazu wird die Sende- und Empfangsvorrichtung mehrfach ausgelegt.

In einer alternativen Variante des oben genannten Ausführungsbeispiels wird ein in ein Funktionsbett bzw. einen Operationstisch integriertes Messsystem zur Erfassung der Atmungsanstrengung als kontinuierliches Signal bereitgestellt. Dabei kann wiederum die Atmungsanstrengung an mindestens einer, aber auch an mehreren Körperpartien gemessen werden, wobei die Sende- und Empfangsvorrichtung bei mehreren Körperpartien mehrfach ausgelegt ist.

In einem weiteren Ausführungsbeispiel schafft die Erfindung ein medizinisches System zum Überwachen der Vitalparameter eines Lebewesens, insbesondere der Atmungsanstrengung, der Atmungsfrequenz, der Atmungsamplitude und des Atmungsvolumens.

Weitere Ausführungsbeispiele der vorliegenden Erfindung schaffen ein Fahrerassistenzsystem zur medizinischen Überwachung des Gesundheitszustandes des Fahrers, insbesondere der Atmungsanstrengung, der Atmungsfrequenz, der Atmungsamplitude und des Atmungsvolumens.

Ausführungsbeispiele stellen ferner ein in den Sitz und den Sicherheitsgurt integriertes Messsystem zum Erfassen der Atmungsanstrengung als kontinuierliches Signal.

Weitere Ausführungsbeispiele können auch in anderen Einrichtungen oder Vorrichtungen angebracht oder integriert werden.

Weitere Ausführungsbeispiele weisen anstelle der induktiven Kopplung eine elektromagnetische Kopplung zwischen dem aktiven Sender und dem Messfühler auf. Dabei können der aktive Sender 10 und der Messfühler 30 jeweils zumindest eine Dipol- oder Patch-Antenne 230, 310 aufweisen und elektromagnetisch miteinander gekoppelt sein, so dass das bereitgestellte Signal von einer durch die Abstandsänderung bedingten Feldstärkeänderung in dem Messfühler abhängt. Anstelle der Dipol- oder Patch-Antenne können der aktive Sender und/oder der Messfühler allgemein Antennen für UHF-(Ultrahochfrequenz), Mikrometer- oder Millimeterwellenbereiche aufweisen.

Bei weiteren Ausführungsbeispielen können der Transponder oder der Messfühler direkt am Körper, z.B. in Form eines Pflasters in dem der Transponder integriert ist, angebracht werden, und so eine Messung auch bei einer Drehung des Körpers ermöglichen. Aufgrund des direkten Kontaktes mit dem Körper des Patienten wird eine höhere Wirkung und/oder Genauigkeit der Messung ermöglicht, und zu dem auch eine Personalisierung, z.B. in Bezug auf spezifische Normwerte oder Alarmfunktionen für den Patienten.

In weiteren Ausführungsbeispielen können der Messfühler oder Transponder auch in Kleidungsstücke integriert werden.

Die Erfindung betrifft damit sowohl ein medizinisches System zum Überwachen der Vitalparameter des Menschen, insbesondere der Atmungsanstrengung, als auch ein Verfahren und eine Vorrichtung zum Erfassen der Atmungsbewegungen am Körper eines Lebewesens allgemein, d. h. zum Beispiel von Menschen, Tieren etc.

Das Anwendungsgebiet der Ausführungsbeispiele der vorliegenden Erfindung liegt beispielsweise im Bereich der präventiven, überwachenden und begleitenden Medizin. Eine direkte Anwendung ist beispielsweise in der Erfassung der Atmungsanstrengung in der Somnologie, Sportmedizin und Homecare (Monitoring des Patienten in der häuslichen Umgebung) möglich.

Abhängig von den Gegebenheiten können die Ausführungsbeispiele der erfindungsgemäßen Verfahren in Hardware oder in Software implementiert werden. Die Implementierung kann auf einem digitalen Speichermedium, insbesondere einer Diskette, CD oder DVD mit elektronisch auslesbaren Steuersignalen erfolgen, die so mit einem programmierbaren Computersystem zusammenwirken, dass eines der Ausführungsbeispiele der erfindungsgemäßen Verfahren ausgeführt wird. Allgemein bestehen die Ausführungsbeispiele der vorliegenden Erfindung somit auch in Software-Programm-Produkten bzw. Computer-Programm-Produkten bzw. Programm-Produkten mit einem auf einem maschinenlesbaren Träger gespeicherten Programmcode zur Durchführung eines der Ausführungsbeispiele der erfindungsgemäßen Verfahren, wenn eines der Softwareprogramm-Produkte auf einem Rechner oder auf einem Prozessor abläuft. In anderen Worten ausgedrückt, kann ein Ausführungsbeispiel der vorliegenden Erfindung somit als ein Computer-Programm bzw. Software-Programm bzw. Programm mit einem Programmcode zur Durchführung eines Ausführungsbeispiel eines erfindungsgemäßen Verfahrens realisiert werden, wenn das Programm auf einem Prozessor abläuft.

Der Prozessor kann hierbei von einem Computer, einer ChipKarte, einem digitalen Signalprozessor oder einem anderen integrierten Schaltkreis gebildet sein.

## Patentansprüche

1. Vorrichtung (100) zum Erfassen einer Atmung eines Lebewesens mit folgenden Merkmalen:
einem aktiven Sender (10), der ausgebildet ist, um ein magnetisches oder elektromagnetisches Feld zu erzeugen;
einem Messfühler (30), der am Rumpf (2) des Lebewesens angeordnet ist und ausgebildet ist, um ein Signal bereitzustellen, das von dem magnetischen oder elektromagnetischen Feld und von einer durch die Atmung des Lebewesens bedingten Abstandsänderung zwischen dem aktiven Sender und dem Messfühler abhängt; und
einer ersten Einrichtung (110) und einer zweiten Einrichtung (130), wobei die erste Einrichtung (110) und die zweite Einrichtung (130) derart ausgebildet und miteinander verbunden sind, und der aktive Sender (10) derart in oder an der ersten Einrichtung (110) angeordnet ist und der Messfühler (30) derart in oder an der zweiten Einrichtung (130) angeordnet ist, dass der aktive Sender (10) und der Messfühler (30) unabhängig von einer Drehung des Rumpfs (2) des Lebewesens auf gegenüberliegenden Seiten des Rumpfs (2) angeordnet sind.

2. Vorrichtung nach Anspruch 1, wobei der aktive Sender (10) und der Messfühler (30) jeweils eine Antenne oder ein Antennensystem (230, 310) aufweisen.

3. Vorrichtung nach Anspruch 2, wobei der aktive Sender (10) und der Messfühler (30) jeweils zumindest eine Antennenspule (230, 310) aufweisen und induktiv miteinander gekoppelt sind, so dass das bereitgestellte Signal von einer durch die Abstandsänderung bedingten Induktionsänderung in dem Messfühler abhängt.

4. Vorrichtung nach Anspruch 2, wobei der aktive Sender (10) und der Messfühler (30) jeweils zumindest eine Antenne (230, 310) für einen UHF- (Ultrahochfrequenz), Mikrometer- oder Millimeterwellenbereich aufweisen und elektromagnetisch miteinander gekoppelt sind, so dass das bereitgestellte Signal von einer durch die Abstandsänderung bedingten Feldstärkeänderung in dem Messfühler abhängt.

5. Vorrichtung nach einem der Ansprüche 1 bis 4, wobei der aktive Sender (10) und der Messfühler (30) ausgebildet sind, um auf gegenüberliegenden Seiten des Rumpfes (2) des Lebewesens angeordnet zu werden.

6. Vorrichtung nach einem der Ansprüche 1 bis 5, wobei die erste Einrichtung (110) oder die zweite Einrichtung (130) eine Rückenlehne eines Sitzes oder Autositzes ist.

7. Vorrichtung nach einem der Ansprüche 1 bis 5, wobei die erste Einrichtung (110) oder die zweite Einrichtung (130) eine Liegefläche ist.

8. Vorrichtung nach Anspruch 6 oder 7, wobei die zweite Einrichtung (130) oder die erste Einrichtung (110) ein Gurt oder Sicherheitsgurt ist.

9. Vorrichtung nach einem der Ansprüche 1 bis 8, wobei weder die erste Einrichtung (110) noch die zweite Einrichtung (130) ein Kleidungsstück ist oder mit dem Körper fest verbunden ist.

10. Vorrichtung nach einem der Ansprüche 1 bis 9, ferner mit folgenden Merkmalen:
einer Auswerteeinheit, die ausgebildet ist, um basierend auf dem bereitgestellten Signal eine Atmungsfrequenz, Atmungsamplitude und/oder ein Atmungsvolumen der Atmung zu bestimmen.

11. Vorrichtung nach Anspruch 10, wobei der Messfühler (30) und die Auswerteeinheit über eine drahtgebundene oder drahtlose Kommunikationsschnittstelle miteinander verbunden sind, um das bereitgestellte Signal von dem Messfühler zu der Auswerteeinheit zu übertragen.

12. Vorrichtung nach Anspruch 11, wobei der Messfühler (30) ein Transponder ist und die drahtlose Kommunikationsschnittstelle auf einem Lastmodulationsverfahren oder einem Backscatter-Verfahren basiert.

13. Verfahren zum Erfassen einer Atmung eines Lebewesens mit folgenden Schritten:
Erzeugen eines magnetischen oder elektromagnetischen Feldes mittels eines aktiven Senders (10); und
Bereitstellen eines Signals mittels eines Messfühlers (30), der am Rumpf des Lebewesens angeordnet ist, wobei das Signal von dem magnetischen oder elektromagnetischen Feld und von einer durch die Atmung des Lebewesens bedingten Abstandsänderung zwischen dem aktiven Sender und dem Messfühler abhängt, wobei der aktive Sender (10) derart in oder an einer ersten Einrichtung (110) angeordnet ist und der Messfühler (30) derart in oder an einer zweiten Einrichtung (130), die mit der ersten Einrichtung (110) verbunden ist, angeordnet ist, dass der aktive Sender (10) und der Messfühler (30) unabhängig von einer Drehung des Rumpfs (2) des Lebewesens auf gegenüberliegenden Seiten des Rumpfs (2) angeordnet sind.

14. Messsystem (100) zum Erfassen einer Atmung einer Person in einem Kraftfahrtzeug, mit folgenden Merkmalen:
einem aktiven Sender (10), der in eine Rückenlehne eines Autositzes des Kraftfahrzeugs integriert ist, und der ausgebildet ist, um ein magnetisches oder elektromagnetisches Feld zu erzeugen; und
einem Messfühler (30), der in einem Sicherheitsgurt des Kraftfahrzeugs in einem angeschnallten Zustand in Höhe des Rumpfs (2) der Person angeordnet ist und der ausgebildet ist, um ein Signal bereitzustellen, das von dem magnetischen oder elektromagnetischen Feld und von einer durch die Atmung des Lebewesens bedingten Abstandsänderung zwischen dem aktiven Sender (10) und dem Messfühler (30) abhängt.

## Claims

1. A device (100) for sensing respiration of a living being, comprising:
an active transmitter (10) configured to generate a magnetic or electromagnetic field;
a sensor (30) arranged on the torso (2) of the living being and configured to provide a signal which depends on the magnetic or electromagnetic field and on a change in distance, caused by the respiration of the living being, between the active transmitter and the sensor; and
a first means (110) and a second means (130), the first means (110) and the second means (130) being configured and connected to each other in such a manner, and the active transmitter (10) being arranged in or on the first means (110) in such a manner, and the sensor (30) being arranged in or on the second means (130) in such a manner, that the active transmitter (10) and the sensor (30) are arranged on opposite sides of the torso (2), irrespectively of a turn of the torso (2) of the living being.

2. The device as claimed in claim 1, wherein the active transmitter (10) and the sensor (30) each comprise an antenna or an antenna system (230, 310).

3. The device as claimed in claim 2, wherein the active transmitter (10) and the sensor (30) each comprise at least one antenna coil (230, 310) and are inductively coupled to each other, so that the provided signal depends on a change in induction within the sensor which is caused by a change in distance.

4. The device as claimed in claim 2, wherein the active transmitter (10) and the sensor (30) each comprise at least one antenna (230, 310) for UHF (ultra-high frequency), micrometer or millimeter wave ranges and are electromagnetically coupled to each other, so that the provided signal depends on a change in field strength within the sensor which is caused by a change in distance.

5. The device as claimed in any one of claims 1 to 4, wherein the active transmitter (10) and the sensor (30) are configured to be arranged on opposite sides of the torso (2) of the living being.

6. The device as claimed in any one of claims 1 to 5, wherein the first means (110) or the second means (130) is a backrest of a seat or car seat.

7. The device as claimed in any one of claims 1 to 5, wherein the first means (110) or the second means (130) is a lying surface.

8. The device as claimed in claim 6 or 7, wherein the second means (130) or the first means (110) is a belt or a safety belt.

9. The device as claimed in any one of claims 1 to 8, wherein neither the first means (110) nor the second means (130) is a piece of clothing or is firmly connected to the body.

10. The device as claimed in any one of claims 1 to 9, further comprising:
an evaluation unit configured to determine, on the basis of the provided signal, a breathing rate, a breathing amplitude, and/or a breathing volume of the respiration.

11. The device as claimed in claim 10, wherein the sensor (30) and the evaluation unit are connected to each other via a wired or wireless communication interface so as to transmit the provided signal from the sensor to the evaluation unit.

12. The device as claimed in claim 11, wherein the sensor (30) is a transponder, and the wireless communication interface is based on a load modulation method or a backscattering method.

13. A method of sensing respiration of a living being, comprising:
generating a magnetic or electromagnetic field by means of an active transmitter (10); and
providing a signal by means of a sensor (30) arranged on the torso of the living being, the signal depending on the magnetic or electromagnetic field and on a change in distance, caused by the respiration of the living being, between the active transmitter and the sensor, the active transmitter (10) being arranged in or on the first means (110) in such a manner, and the sensor (30) being arranged in or on the second means (130) connected to the first means (110) in such a manner, that the active transmitter (10) and the sensor (30) are arranged on opposite sides of the torso (2), irrespectively of a turn of the torso (2) of the living being.

14. A measuring system (100) for sensing respiration of a person situated within a motor vehicle, comprising:
an active transmitter (10) integrated into a backrest of a car seat of the motor vehicle and configured to generate a magnetic or electromagnetic field; and
a sensor (30) configured to be arranged within a safety belt of the motor vehicle, in a state in which the safety belt is fastened, at the height of the person's torso (2), and configured to provide a signal which depends on the magnetic or electromagnetic field and on a change in distance, caused by the respiration of the living being, between the active transmitter (10) and the sensor (30).

## Revendications

1. Dispositif (100) de détermination d'une respiration d'un être vivant, aux caractéristiques suivantes:
un émetteur actif (10) qui est conçu pour générer un champ magnétique ou électromagnétique;
un capteur de mesure (30) disposé sur le ventre (2) de l'être vivant et conçu pour mettre à disposition un signal qui dépend du champ magnétique ou électromagnétique et d'une variation de distance entre l'émetteur actif et le capteur de mesure résultant de la respiration de l'être vivant; et
un premier moyen (110) et un deuxième moyen (130), le premier moyen (110) et le deuxième moyen (130) étant conçus et connectés l'un à l'autre et l'émetteur actif (10) étant disposé dans ou sur le premier moyen (110) et le capteur de mesure (30) étant disposé dans ou sur le deuxième moyen (130) de sorte que l'émetteur actif (10) et le capteur de mesure (30) soient disposés sur des côtés opposés du ventre (2) indépendamment d'une rotation du ventre (2) de l'être vivant.

2. Dispositif selon la revendication 1, dans lequel l'émetteur actif (10) et le capteur de mesure (30) présentent, chacun, une antenne ou un système d'antennes (230, 310).

3. Dispositif selon la revendication 2, dans lequel l'émetteur actif (10) et le capteur de mesure (30) présentent, chacun, au moins une bobine d'antenne (230, 310) et sont couplés l'un à l'autre de manière inductive, de sorte que le signal mis à disposition dépende d'une variation d'induction dans le capteur de mesure résultant de la variation de distance.

4. Dispositif selon la revendication 2, dans lequel l'émetteur actif (10) et le capteur de mesure (30) présentent, chacun, au moins une antenne (230, 310) pour une plage d'ondes UHF (ultra hautes fréquences), micrométriques ou millimétriques et sont couplés l'un à l'autre de manière électromagnétique, de sorte que le signal mis à disposition dépende d'une variation d'intensité de champ dans le capteur de mesure résultant d'une variation de distance.

5. Dispositif selon l'une des revendications 1 à 4, dans lequel l'émetteur actif (10) et le capteur de mesure (30) sont conçus pour être disposés sur des côtés opposés du ventre (2) de l'être vivant.

6. Dispositif selon l'une des revendications 1 à 5, dans lequel le premier moyen (110) ou le deuxième moyen (130) est un dossier d'un siège ou d'un siège de voiture.

7. Dispositif selon l'une des revendications 1 à 5, dans lequel le premier moyen (110) ou le deuxième moyen (130) est une surface de couchage.

8. Dispositif selon la revendication 6 ou 7, dans lequel le deuxième moyen (130) ou le premier moyen (110) est une sangle ou ceinture de sécurité.

9. Dispositif selon l'une des revendications 1 à 8, dans lequel ni le premier moyen (110) ni le deuxième moyen (130) est un vêtement ou est relié fermement au corps.

10. Dispositif selon l'une des revendications 1 à 9, par ailleurs aux caractéristiques suivantes:
une unité d'évaluation qui est conçue pour déterminer, sur base du signal mis à disposition, un rythme, une amplitude et/ou un volume de la respiration.

11. Dispositif selon la revendication 10, dans lequel le capteur de mesure (30) et l'unité d'évaluation sont connectés l'un à l'autre par l'intermédiaire d'une interface de communication filaire ou sans fil, pour transmettre le signal mis à disposition du capteur de mesure à l'unité d'évaluation.

12. Dispositif selon la revendication 11, dans lequel le capteur de mesure (30) est un transpondeur et l'interface de communication sans fil est basée sur un procédé de modulation de charge ou un procédé de rétrodiffusion.

13. Procédé de détermination d'une respiration d'un être vivant, aux étapes suivantes consistant à:
générer un champ magnétique ou électromagnétique au moyen d'un émetteur actif (10); et
mettre à disposition un signal au moyen d'un capteur de mesure (30) qui est disposé sur le ventre de l'être vivant, où le signal dépend du champ magnétique ou électromagnétique et d'une variation de distance entre l'émetteur actif et le capteur de mesure résultant de la respiration de l'être vivant, où l'émetteur actif (10) est disposé dans ou sur un premier moyen (110) et le capteur de mesure (30) est disposé dans ou sur un deuxième moyen (130) qui est relié au premier moyen (110), de sorte que l'émetteur actif (10) et le capteur de mesure (30) soient disposés sur des côtés opposés ventre (2) indépendamment d'une rotation du ventre (2) de l'être vivant.

14. Système de mesure (100) pour déterminer une respiration d'une personne dans un véhicule automobile, aux caractéristiques suivantes:
un émetteur actif (10) qui est intégré dans un dossier d'un siège de voiture du véhicule automobile et qui est conçu pour générer un champ magnétique ou électromagnétique; et
un capteur de mesure (30) qui est disposé dans une ceinture de sécurité du véhicule automobile à l'état attaché à hauteur du ventre (2) de la personne et qui est conçu pour mettre à disposition un signal qui dépend du champ magnétique ou électromagnétique et d'une variation de distance entre l'émetteur actif (10) et le capteur de mesure (30) résultant de la respiration de l'être vivant.
